# EUROPEAN PATENT SPECIFICATION

(11) **EP 2 653 132 B1**
(45) Date of publication and mention of the grant of the patent: **04.11.2020**
(21) Application number: 13159655.3
(22) Date of filing: 17.03.2013
(51) Int. Cl.: A61F 2/00

(54) **Incontinence treatment device including non-porous cuff and extending members**
Inkontinenzbehandlungsvorrichtung mit nichtporöser Stulpe und Erweiterungselementen
Dispositif de traitement de l'incontinence comprenant un ballonnet non poreux et des éléments d'extension

(30) Priority: 20.04.2012 DK 201270202; 23.04.2012 US 201213453064; 14.11.2012 US 201213676132
(43) Date of publication of application: 23.10.2013
(73) Proprietor: Coloplast A/S, 3050 Humlebaek (DK)
(72) Inventor: Deegan, Christopher, North St. Paul, MN 55109 (US); McClurg, Steven, Brooklyn Park, MN 55444 (US); Moschel, Mark A., New Hope, MN 55427 (US)

(56) References cited:
- US-A- 5 860 911
- US-A1- 2004 173 219
- US-A1- 2006 264 697
- US-A1- 2011 077 455

## Description

### Background

Devices for treating urinary incontinence include slings, supports, artificial urinary sphincters and other devices that are implanted in a user to support and/or coapt the urethra.

A sling is a device that is surgically implanted to support the urethra and inhibit urine from undesirably leaking from the urethra. Slings are typically fabricated from mesh and are implanted through one or more incisions. The sling is secured to supporting tissue(s) and the tissue eventually grows through the mesh to support the urethra. The surgeon will peri-operatively determine and achieve the appropriate level of tension in the sling relative to the urethra that will postoperatively provide the user with a continent state.

An artificial urinary sphincter is generally provided as an inflatable ring or "donut" that is surgically implanted around the urethra. Some people become incontinent after having a portion or all of the prostate gland removed, which can result in a loss of some or all of the function of the prostatic urinary sphincter. An artificial urinary sphincter implanted around the compromised prostatic urinary sphincter can provide the patient with improved control of urinary function.

US 2006/0264697A1 discloses an implantable incontinence device and method providing for occluding the urethra in preventing urine leakage. The device includes an occluding member connected to a control mechanism. The occluding member is movable from a contracted condition to an expansive condition by activating and deactivating the control mechanism.

US5860911 discloses a device for control of masculine incontinence including a single laminar body constituted by a layer of spongy material covered on one of its faces by a sheet of semi-rigid synthetic material, constituting two successive curved areas separated from each other by a hinge fold end ending in individual flat flanges capable of coinciding when the device is folded.

US 2011/0077455A1 discloses a medical reinforcement graft for reinforcing patient tissue structures and methods of making and using the graft device. The graft devices can have a remodelable extracellular matrix material graft body defining a slot and having a portion receivable through the slot to form a closed loop.

US 2004/0173219 discloses an external male urinary incontinence device for location around the shaft of a penis.

Improved incontinence treatment devices would be welcomed by both the patient and the surgical staff.

### Summary

One aspect provides an incontinence treatment device including a non-porous support and an extending member. The non-porous support has a first end portion provided with a slot and a second end portion. The second end portion is insertable through the slot to form a cuff that is configured for placement around a urethra of a user. The extending member is attached to the support at a junction located between the first end portion and the second end portion. Tension applied to the second end portion is adapted to draw the junction toward the slot to tighten the cuff and thus provide the user with a continent state. Tension applied to the extending member is adapted to draw the juncture away from the slot and expand the cuff and thus allow the user to urinate.

One aspect provides an incontinence treatment device including a non-porous sheet and an extending member attached to the non-porous sheet. The non-porous sheet has a first side spaced apart from a second side by a sheet width, a first end portion having a width that is approximately equal to the sheet width with a slot formed in the first end portion, and a second end portion that tapers to a tapered width that is less than the sheet width. The extending member has a first end that is attached to the sheet at a junction located between the first end portion and the second end portion. The extending member includes a second end that has a width that is less than the sheet width. The tapered width of the second end portion of the sheet is insertable through the slot in the first end portion to form a cuff from the non-porous sheet that is sized for placement around a human urethra. The slot has a slot width that prevents the junction from passing through the slot.

One aspect provides an example of a method of treating urinary incontinence that includes making an incision and exposing tissue of a urethra, and inserting a non-porous sheet into the incision and forming a cuff around the urethra with the non-porous sheet by directing a first end of the non-porous sheet through a slot formed in a second end of the non-porous sheet. The method further includes directing an extending member that is attached to the non-porous sheet to a location outside of the pelvis. The method additionally includes configuring the cuff to tighten around the urethra when pulling on the first end of the non-porous sheet, and configuring the cuff to be loosely positioned around the urethra when pulling on the extending member.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a top view of one embodiment of an incontinence treatment device.
Figure 2 is a perspective view of the incontinence treatment device illustrated in Figure 1 as assembled to provide a cuff.
Figure 3 is a top view of the incontinence treatment device illustrated in Figure 2.
Figure 4A is a top view and Figure 4B is an expanded view of the incontinence treatment device illustrated in Figure 3 with tension applied to tighten the cuff of the device.
Figure 4C is a top view of the incontinence treatment device illustrated in Figure 4A with tension applied to a second extending member to loosen the cuff of the device.
Figure 5 is a schematic view of one embodiment of an incontinence treatment device with a cuff implanted around the urethra and extending members directed behind a pubic symphysis and through a wall of an abdomen.
Figure 6 is a schematic view of one embodiment of an incontinence treatment device with a cuff implanted around the urethra and extending members directed through obturator foramina and through a groin of a user.
Figure 7A is a schematic view of one embodiment of an incontinence treatment device with a cuff loosely implanted around the urethra and extending members implanted in a scrotum.
Figure 7B is a schematic view of one embodiment of the incontinence treatment device illustrated in Figure 7A with the cuff tightened around the urethra to provide the user with a continent state.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Tissue includes soft tissue, which includes dermal tissue, sub-dermal tissue, ligaments, tendons, or membranes. As employed in this specification, the term "tissue" does not include bone.

In this specification the word "coapt" means to close or to substantially close. To coapt an opening is to bring two surfaces together in close approximation such that the gap between the two surfaces is reduced or eliminated. To coapt a urethra means to substantially yet reversibly close the urethra to impede the passage of urine and provide a person with a continent state.

In this specification permanent means for as long as an incontinence treatment device is implanted into a patient. Thus, an incontinence treatment device having a permanent external portion outside of an abdominal wall of the patient provides the external portion outside of the patient for as long as the device is implanted in the patient.

In this specification a "user" is one into whom an incontinence treatment device has been implanted.

In this specification "non-porous" means a material with no through-holes. For example, a non-porous material is characterized by the absence of multiple holes that extend through the material from one surface to the other surface. Non-porous, as employed in this specification, means a material that is configured to prevent tissue growth through the implanted material.

Embodiments provide an incontinence treatment device implantable into a user and including extending members that allow the user to selectively move from a state of continence (free from urine leakage) to a state that allows urination. Tension applied to a first extending member is adapted to draw the junction toward the slot to tighten the cuff and coapt the urethra to provide the user with a continent state. Tension applied to the second extending member is adapted to draw the juncture away from the slot and loosen the cuff to relieve coaptation of the urethra and allow the user to urinate.

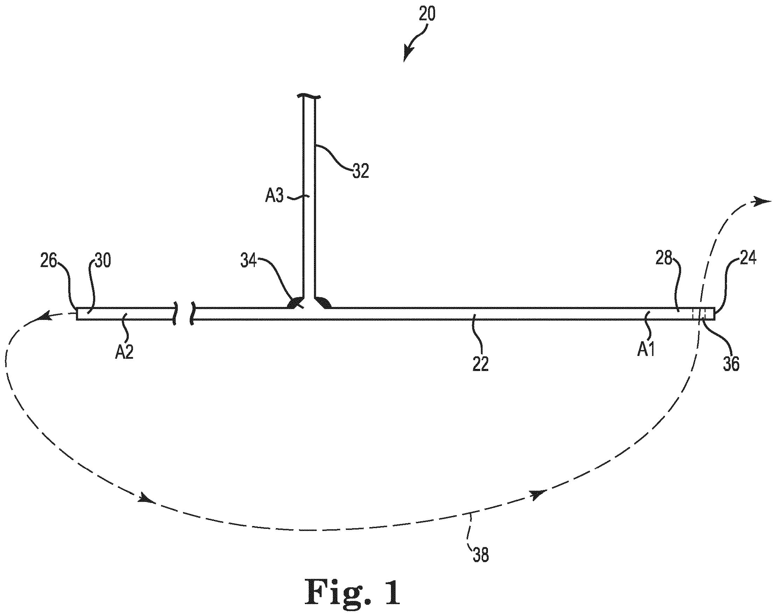

Figure 1 is a top view of one embodiment of an incontinence treatment device 20. The incontinence treatment device 20 (device 20) includes a support 22 in the form of a sheet or a band that extends between a first end 24 and a second end 26, with a first end portion 28 adjacent to the first end 24 and a second end portion 30 adjacent to the second end 26. An extending member 32 is attached to the support 22 at a junction 34 that is located between the first end portion 28 and the second end portion 30. The support 22 is fabricated to be non-porous such that after implantation the non-porous support 22 discourages or prevents tissue from growing onto or through the non-porous support 22.

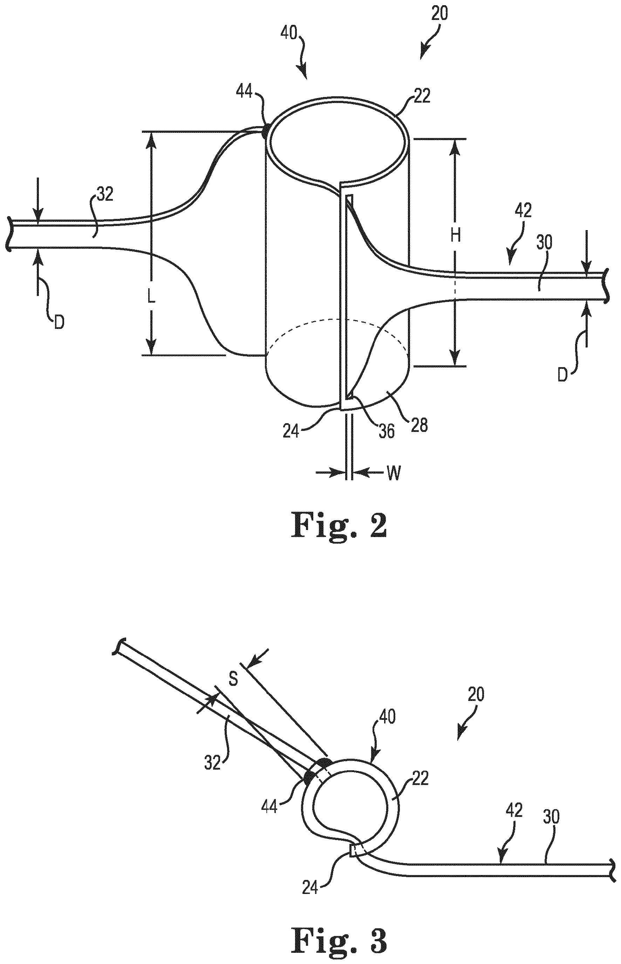

One of the end portions 28, 30 is provided with a slot 36. For example, the first end portion 28 is provided with a slot 36 that is sized to receive the second end portion 30. Inserting the second end portion 30 through the slot 36, for example along a path 38, forms the support 22 into a cuff (40 in Figure 2) with the second end portion 30 extending in a different direction from the extending member 32.

Prior to assembly, the device 20 provides three segments or arms: a first arm A1 that is provided by the first end portion 28 having the slot 36, a second arm A2 that is provided by the second end portion 30, and a third arm A3 that is provided by the second extending member 32.

In one embodiment, the second extending member 32 is attached at a substantially orthogonal angle to the support 22. In one embodiment, the second extending member 32 is attached to the support 22 at an angle other than 90 degrees.

Figure 2 is a perspective view and Figure 3 is a top view of the support 22 providing a cuff 40. The cuff 40 has two adjustment arms, a tightening arm provided by the second end portion 30 and a loosening arm provided by extending member 32.

During implantation, the support 22 is placed on a first side of the urethra and the second end portion 30 is inserted through the slot 36 to form the cuff 40 around the urethra of a user. The assembled cuff 40 thus has two extending members: a first extending member 42 provided by the second end portion 30 and the second extending member 32 attached to the support 22 between the first end portion 28 and the second end portion 30.

In one embodiment, the slot 36 is formed to have a slot width W (Figure 2). In one embodiment, the junction 34 between the member 32 and the support 22 includes a stopper 44 with a stop width S that is larger than the slot width W. The stop width S of the stopper 44 is configured to prevent the junction 34 from passing through the slot 36. In one embodiment, the slot width W is sized to prevent passage of the extending member 32 through the slot 36.

In one embodiment, the support 22 has a height H extending between opposing sides of the support 22, and each of the first extending member 42 and the second extending member 32 tapers from a first width L that is substantially equal to the height H of the support 22 to a second width D that is narrower than the height H of the support.

The support 22 is non-porous and is preferably fabricated from a synthetic material such as rubber, silicone, thermoplastic polymer, thermoset polymer, or blends of polymers or copolymers. One suitable material for fabricating the support 22 is silicone rubber. The support 22 is fabricated to be non-porous to discourage tissue growth through the surface of the support 22.

In one embodiment, at least the cuff 40 portion of the device 20 is coated with a substance that prevents tissue from attaching to or growing on a surface of the cuff 40. Tissue growth into or through the support 22 would undesirably fixate the support 22 into the patient after it is implanted. The support 22 is fabricated from materials that are non-porous and are selected to reduce or prevent the likelihood of tissue growth into or through the support 22. Although not bound to this theory, tissue is expected to grow around the cuff 40 and envelop the cuff 40 in a pocket of tissue that allows the cuff 40 to move (tighten and loosen) relative to the urethra.

In one embodiment, since the non-porous support 22 is not a mesh. In one embodiment, the non-porous support 22 has a thickness and is fabricated from, for example, a solid film having substantially uniform density through the thickness. Thus, in one embodiment, the support 22 and the resulting assembled cuff 40 are not inflatable.

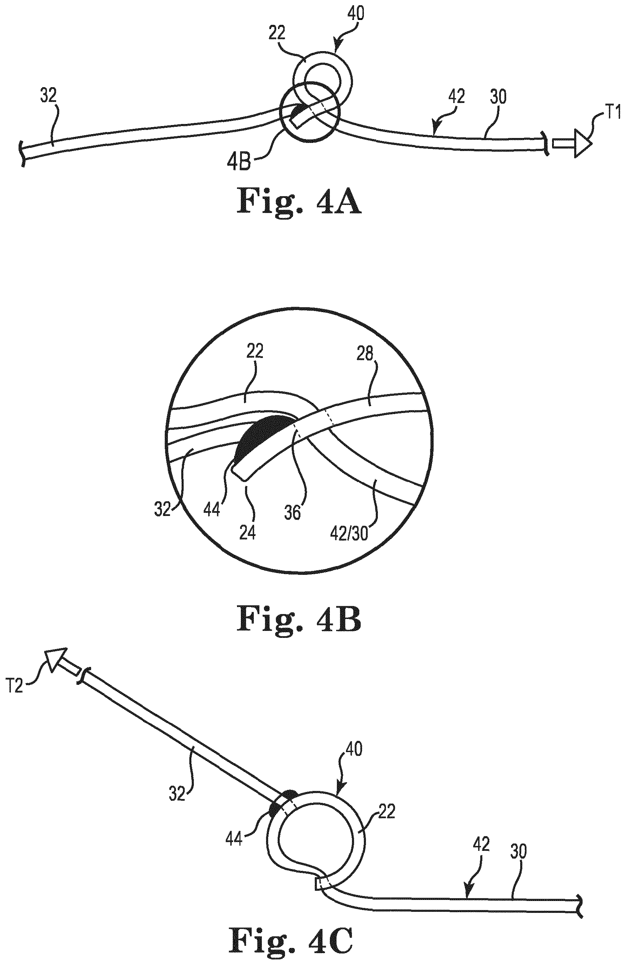

Figure 4A is a top view and Figure 4B is an expanded view of the cuff 40. Tension T1 illustrates a pulling force applied to the first extending member 42. The tension T1 will draw the junction 34 up to the slot 36 and reduce the diameter of the cuff 40, which is useful to coapt the urethra to provide the user with a continent state. With reference to Figure 4B, the tension T1 applied to the first extending member 42 tightens the cuff 40 until a stopper 44 is caught by the slot 36, which prevents further collapse of the cuff 40.

The arc length of the support 22 extending from the junction 34 to the slot 36 (See Figure 1) is selected such that the cuff 40 provides an inside diameter that is sufficient to collapse or coapt a human urethra without over compressing or eroding the urethra. The stopper 44 thus provides a feature that prevents the cuff 40 from being collapsed too tightly against the urethra.

Figure 4C is a top view illustrating a second tension T2 applied to the second extending member 32 to draw the stopper 44 away from the slot 36, which expands the cuff 40 to relieve coaptation of the urethra to allow the user to urinate.

When implanted, the cuff 40 provides the user with the ability to pull on the first extending member 42 to achieve a continent state, and to subsequently pull on the second extending member 32 to transition the user into a state that allows urine to pass through the urethra.

In one embodiment, the cuff 40 is user-activated with each of the first and second extending members 42, 32, respectively, implanted to be accessible to the user such that user-applied tension T1 applied to the first extending member 42 will coapt the urethra to provide the user with a continent state, and user-applied tension T2 applied to the second extending member 32 will relieve the coaptation of the urethra and allow the user to urinate.

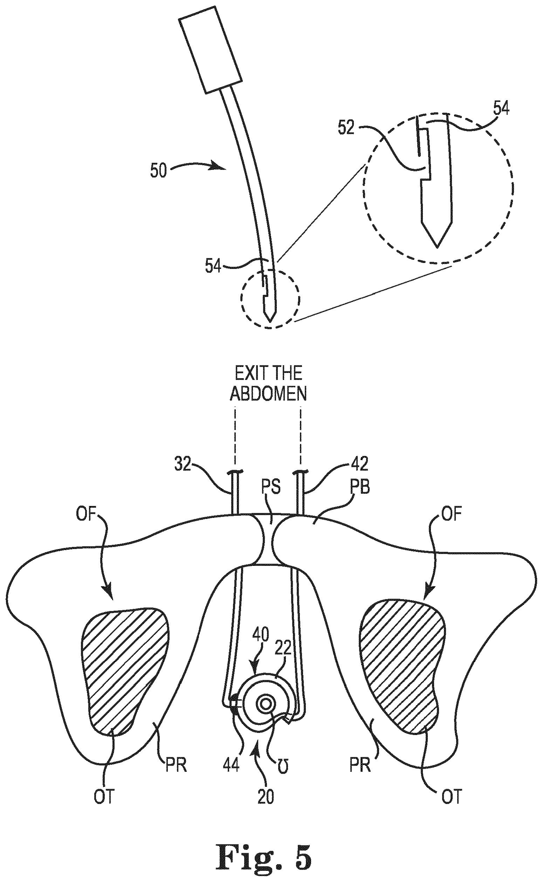

Figure 5 is a schematic view of one embodiment of the device 20 after implantation. The cuff 40 is implanted around the urethra U and the extending members 32, 42 are tunneled behind the pubic bone PB and project through a wall of the abdomen. The extending members 32, 42 are located in a user-accessible location outside the body. Tension applied by the user to the first extending member 42 tightens the cuff 40 to coapt the urethra and provide the user with a continent state. Tension applied by the user to the second extending member 32 loosens the cuff 40 to relieve the coaptation of the urethra and allow the user to urinate.

In one embodiment, the device 20 is implanted into the patient such that the cuff 40 surrounds the urethra U and the extending members 32, 42 exit through the skin of the abdomen of the user. In one the suitable implantation approach, a perineal incision (male) or a vaginal incision (female) is made, and tissue surrounding the urethra U is dissected to gain access to the urethra U. A portion of the device 20 is inserted into the incision. With reference to Figure 1, the second end portion 30 is subsequently maneuvered around the urethra U and inserted through the slot 36 in the first end portion 28 to form the cuff 40 around the urethra U. A suitable tunneling device, such as a pointed introducer 50 or needle-like device, is inserted through the skin of the abdomen at an access point in an abdominal location that is superior to the pubic symphysis PS. The introducer 50 is tunneled downward toward the urethra U and exits the incision formed in the patient. One of the extending members 32, 42 is engaged with a T-slot 52 formed in an end portion 54 of the introducer 50, and the introducer 50 is retracted upwards behind the pubic bone PB and out of the access point formed in the abdominal location. This maneuver is repeated on the contralateral side, in which the pointed introducer 50 is inserted through the skin and into the abdomen at a second access point that is superior to the pubic symphysis PS and again tunneled downward to exit the incision formed in the patient. The other of the extending members 32, 42 is engaged with the slot 52 of the introducer 50, and the introducer 50 is retracted upwards behind the pubic bone PB and out of the second access point formed in the abdomen.

The perineal or the vaginal incision is closed. The extending members 32, 42 project outward from the abdomen through the access points formed in the abdominal locations. A suitable barrier is provided to prevent the transmission of bacteria into the abdominal locations. In other approaches, a prophylactic antibiotic is employed to reduce infection at the abdominal locations.

The first and second extending members 42, 32, respectively, are thus accessible to the user. Tension applied to the first extending member 42 will tighten the cuff 40 to coapt the urethra U. Tension applied to the second extending member 32 will expand the cuff 40 to allow the user to pass urine through the urethra U.

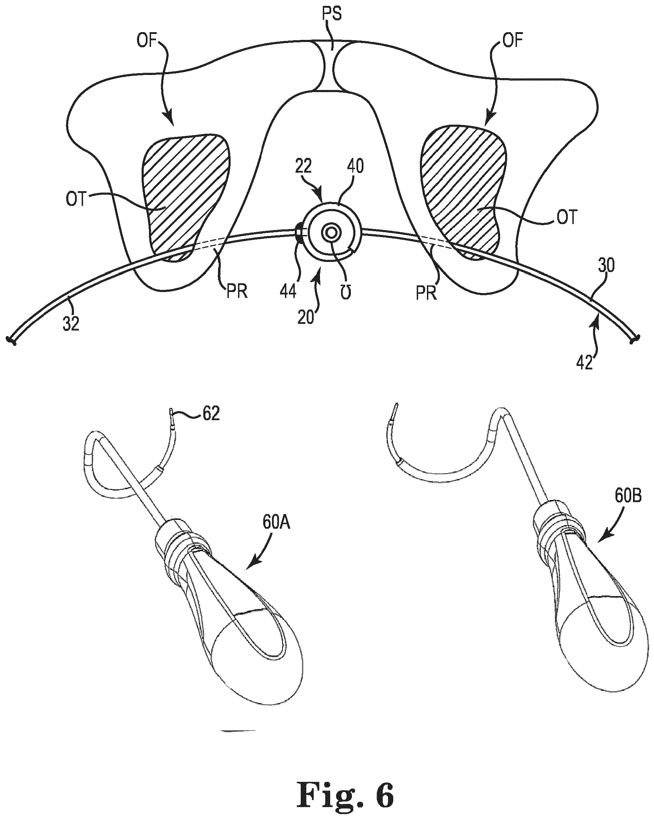

Figure 6 is a schematic view of one embodiment of the cuff 40 implanted around the urethra U with each of the extending members 32, 42 directed through one of the obturator foramina OF and out of the groin to a user-accessible location.

In one embodiment, the device 20 is implanted into the patient such that the cuff 40 surrounds the urethra U and the extending members 32, 42 extend through an obturator foramen and out of the skin of the groin. In one suitable implantation approach, a perineal incision (male) or a vaginal incision (female) is made, and tissue surrounding urethra is dissected to gain access to the urethra U. With reference to Figure 1, the second end portion 30 is maneuvered around the urethra U and inserted through the slot 36 in the first end portion 28 to form the cuff 40 around the urethra U. The ends of the extending members 32, 42 are suitably tunneled through each of the obturator foramen OF and out of the skin in the region of the groin.

In one embodiment, an introducer 60A is employed in an "inside out" approach to tunnel the ends of the extending members 32, 42 out of the skin of the groin. For example, an end of the second extending member 32 is engaged with a tip 62 of the introducer 60A at a location outside of the incision (perineal for male or vaginal for female). The introducer 60A is suitable curved to allow the end of the extending member 32 to be directed behind the descending pubic ramus PR, through one of the obturator foramen OF, and out of the skin in the region of the groin. A similar maneuver is repeated on the contralateral side with a suitably formed second introducer 60B to direct the end of the extending member 42 through the other obturator foramen OF.

Another suitable approach directs an introducer through an "outside in" approach in which the introducer enters the skin in the groin region, penetrates the obturator foramen OF, and is directed around the descending pubic ramus PR until a tip of the introducer exits the incision. The tip of the introducer is coupled with an end of one of the extending members 32, 42, and the introducer is retrieved back through the incision, the obturator foramen OF, and the skin in the groin region. A similar maneuver is repeated on the contralateral side to place the other of the extending members 32, 42.

Thus, as illustrated in Figure 6, each of the first and second extending members 42, 32, respectively, is implanted through one of the obturator foramen and extends to the skin to a user-accessible location.

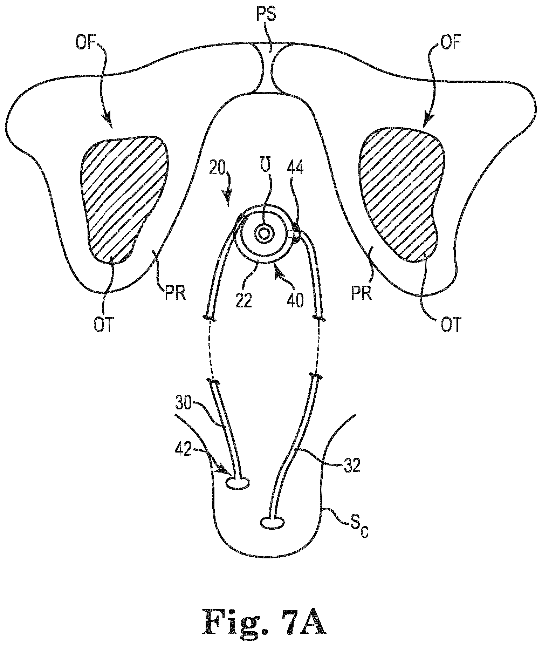

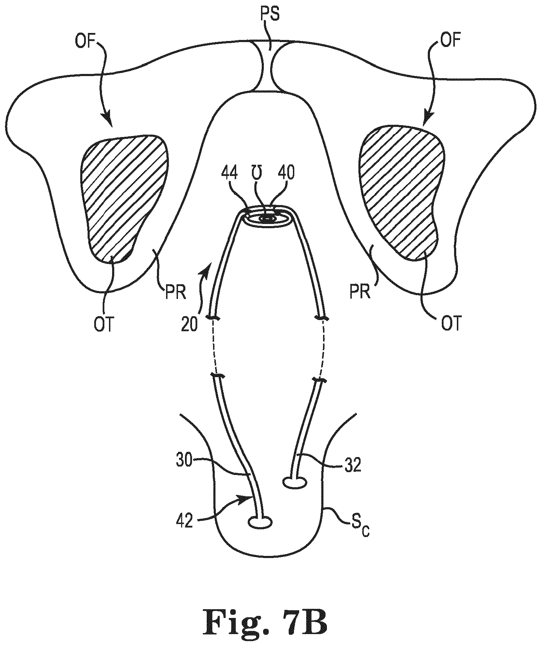

Figure 7A is a schematic view of one embodiment of an open cuff 40 implanted around the urethra U and Figure 7B is a schematic view of the cuff 40 coapting the urethra U.

In one embodiment, the extending members 32, 42 are implanted in a scrotum of a male user, and although implanted internal to the user, are accessible for selectively altering the user between a continent state and a urination state.

Similar to the approaches described above, an incision is made, and tissue surrounding urethra is dissected to gain access to the urethra U. The non-porous sheet 22 is maneuvered around the urethra U to form the cuff 40 around the urethra U. The ends of the extending members 32, 42 are directed into the scrotum Sc and the incision is closed.

The user pulls on the extending members 32, 42 through the skin of the scrotum to adjust the cuff 40 between the tightened and coapted state and the loosened and open states. Figure 7A illustrates the second extending member 32 activated or pulled to open the cuff 40. In this configuration, the user is provided with an opened cuff 40 that allows the user to pass urine through the urethra U.

Figure 7B illustrates the first extending member 42 tensioned or otherwise pulled to collapse the cuff 40 around the urethra U, which provides the user with a continent state.

## Claims

1. An incontinence treatment device (20) comprising:
a non-porous support (22) having a first end portion (28) provided with a slot (36) and a second end portion (30), the second end portion (30) is insertable through the slot (36) to form a cuff (40) that is configured for placement around a urethra of a user; and
an extending member (32) attached to the support (22) at a junction (34) located between the first end portion (28) and the second end portion (30);
wherein tension applied to the second end portion (30) is adapted to draw the junction (34) toward the slot (36) to tighten the cuff (40) and thus provide the user with a continent state; **characterized in that**
tension applied to the extending member (32) is adapted to draw the junction (34) away from the slot (36) and expand the cuff (40) and thus allow the user to urinate.

2. The incontinence treatment device of claim 1, wherein the support (22) has a height extending between a first side and a second side of the support, and the second end portion (28) and the extending member (32) both taper from a first width that is equal to the height of the support (22) to a second width that is narrower than the height of the support (22).

3. The incontinence treatment device of claim 1, wherein the slot (36) has a slot width and the junction (34) located between the first end portion (28) and the second end portion (30) has a stop width that is larger than the slot width and is so configured to prevent the junction (34) from passing through the slot (36).

4. The incontinence treatment device of claim 1, wherein the support (22) is not inflatable.

5. The incontinence treatment device of claim 1, wherein the support (22) has a thickness and is fabricated from a film having uniform density through the thickness.

6. The incontinence treatment device of claim 1, wherein the cuff (40) is user-activated with each of the second end portion (30) and the extending member (32) is accessible to the user such that user-applied tension applied to the second end portion (30) is adapted to coapt the urethra and provide the user with a continent state, and user-applied tension applied to the extending member (32) is adapted to relieve coaptation of the urethra and allow the user to urinate.

7. The incontinence treatment device of claim 1, wherein the non-porous support (22) has three arms including the first end portion (28) provided with the slot (36), the second end portion (30), and the extending member (36) that is attached to the support (22) at the junction (34).

8. The incontinence treatment device of claim 1, wherein the extending member (36) is attached at a orthogonal angle to the support (22).

9. The incontinence treatment device of claim 1, wherein the cuff (40) is coated with a substance that is configured to prevent tissue attachment to the cuff (40).

10. The incontinence treatment device of claim 1, wherein the non-porous support (22) includes a first side spaced apart from a second side by a sheet width, and the first end portion (28) has a width that is equal to the sheet width and the second end portion (30) tapers to a tapered width that is less than the sheet width.

## Patentansprüche

1. Inkontinenzbehandlungsvorrichtung (20), umfassend:
eine nichtporöse Stütze (22) mit einem ersten Endabschnitt (28), der mit einem Schlitz (36) versehen ist, und einem zweiten Endabschnitt (30), wobei der zweite Endabschnitt (30) durch den Schlitz (36) einsetzbar ist, um eine Stulpe (40) zu bilden, die zur Platzierung rund um eine Harnröhre eines Benutzers ausgelegt ist; und
ein Erweiterungselement (32), das an einer Verbindung (34), die sich zwischen dem ersten Endabschnitt (28) und dem zweiten Endabschnitt (30) befindet, an der Stütze (22) angebracht ist;
wobei Spannung, die auf den zweiten Endabschnitt (30) aufgebracht wird, dazu angepasst ist, die Verbindung (34) in Richtung des Schlitzes (36) zu ziehen, um die Stulpe (40) festzuziehen und so dem Benutzer einen kontinenten Zustand zu verleihen; **dadurch gekennzeichnet, dass**
die Spannung, die auf das Erweiterungselement (32) aufgebracht wird, dazu angepasst ist, die Verbindung (34) von dem Schlitz (36) wegzuziehen und die Stulpe (40) auszudehnen und dem Benutzer das Urinieren zu ermöglichen.

2. Inkontinenzbehandlungsvorrichtung nach Anspruch 1, wobei die Stütze (22) eine Höhe aufweist, die sich zwischen einer ersten Seite und einer zweiten Seite der Stütze erstreckt, und sich der zweite Endabschnitt (28) und das Erweiterungselement (32) beide von einer ersten Breite, die gleich der Höhe der Stütze (22) ist, zu einer zweiten Breite die schmaler als die Höhe der Stütze (22) ist, verjüngen.

3. Inkontinenzbehandlungsvorrichtung nach Anspruch 1, wobei der Schlitz (36) eine Schlitzbreite aufweist und die Verbindung (34), die sich zwischen dem ersten Endabschnitt (28) und dem zweiten Endabschnitt (30) befindet, eine Stoppbreite aufweist, die größer als die Schlitzbreite und dazu ausgelegt ist, zu verhindern, dass die Verbindung (34) durch den Schlitz (36) durchgeht.

4. Inkontinenzbehandlungsvorrichtung nach Anspruch 1, wobei die Stütze (22) nicht aufblasbar ist.

5. Inkontinenzbehandlungsvorrichtung nach Anspruch 1, wobei die Stütze (22) eine Dicke aufweist und aus einer Folie hergestellt ist, die über die gesamte Dicke eine gleichmäßige Dichte aufweist.

6. Inkontinenzbehandlungsvorrichtung nach Anspruch 1, wobei die Stulpe (40) vom Benutzer aktiviert wird, indem der zweite Endabschnitt (30) und das Erweiterungselement (32) jeweils vom Benutzer zugänglich sind, sodass die vom Benutzer aufgebrachte Spannung, die auf den zweiten Endabschnitt (30) aufgebracht wird, dazu angepasst ist, die Harnröhre zu koaptieren und dem Benutzer einen kontinenten Zustand verleihen, und die vom Benutzer aufgebrachte Spannung, die auf das Erweiterungselement (32) aufgebracht wird, dazu angepasst ist, die Koaptation der Harnröhre zu lösen und dem Benutzer das Urinieren zu ermöglichen.

7. Inkontinenzbehandlungsvorrichtung nach Anspruch 1, wobei die nichtporöse Stütze (22) drei Arme aufweist, die den ersten mit dem Schlitz (36) versehenen Endabschnitt (28), den zweiten Endabschnitt (30) und das Erweiterungselement (36), das an der Verbindung (34) an der Stütze (22) angebracht ist, umfassen.

8. Inkontinenzbehandlungsvorrichtung nach Anspruch 1, wobei das Erweiterungselement (36) in einem rechten Winkel an der Stütze (22) angebracht ist.

9. Inkontinenzbehandlungsvorrichtung nach Anspruch 1, wobei die Stulpe (40) mit einer Substanz beschichtet ist, die dazu ausgelegt ist, Gewebeanhaftung an der Stulpe (40) zu verhindern.

10. Inkontinenzbehandlungsvorrichtung nach Anspruch 1, wobei die nichtporöse Stütze (22) eine erste Seite umfasst, die von einer zweiten Seite durch eine Bahnbreite beabstandet ist, und der erste Endabschnitt (28) eine Breite aufweist, die gleich der Bahnbreite ist und sich der zweite Endabschnitt (30) zu einer verjüngten Breite verjüngt, die geringer ist als die Bahnbreite.

## Revendications

1. Dispositif de traitement de l'incontinence (20) comprenant :
un support non poreux (22) possédant une première partie terminale (28) dotée d'une fente (36) et une seconde partie terminale (30), la seconde partie terminale (30) pouvant être introduite à travers la fente (36) afin de former un manchon (40) qui est conçu pour un placement autour d'un urètre d'un utilisateur ; et
un élément d'extension (32) fixé au support (22) au niveau d'une jonction (34) située entre la première partie terminale (28) et la seconde partie terminale (30) ;
dans lequel une tension appliquée à la seconde partie terminale (30) est conçue pour tirer la jonction (34) en direction de la fente (36) afin de serrer le manchon (40) et ainsi d'apporter à l'utilisateur un état de continence ; **caractérisé en ce que**
une tension appliquée à l'élément d'extension (32) est conçue pour tirer la jonction (34) à distance de la fente (36) et pour déployer le manchon (40) et permettre ainsi à l'utilisateur d'uriner.

2. Dispositif de traitement de l'incontinence selon la revendication 1, dans lequel le support (22) a une hauteur s'étendant entre un premier côté et un second côté du support, et la seconde partie terminale (28) et l'élément d'extension (32) s'effilent tous les deux depuis une première largeur qui est égale à la hauteur du support (22) vers une seconde largeur qui est plus étroite que la hauteur du support (22).

3. Dispositif de traitement de l'incontinence selon la revendication 1, dans lequel la fente (36) présente une largeur de fente et la jonction (34) située entre la première partie terminale (28) et la seconde partie terminale (30) possède une largeur de butée qui est plus grande que la largeur de fente et est configurée de la sorte pour empêcher que la jonction (34) passe à travers la fente (36).

4. Dispositif de traitement de l'incontinence selon la revendication 1, dans lequel le support (22) n'est pas gonflable.

5. Dispositif de traitement de l'incontinence selon la revendication 1, dans lequel le support (22) a une certaine épaisseur et est fabriqué à partir d'un film ayant une densité uniforme à travers l'épaisseur.

6. Dispositif de traitement de l'incontinence selon la revendication 1, dans lequel le manchon (40) est activé par l'utilisateur, chacun de la seconde partie terminale (30) et de l'élément d'extension (32) étant accessible à l'utilisateur de sorte qu'une tension appliquée par l'utilisateur à la seconde partie terminale (30) soit conçue pour coapter l'urètre et apporter à l'utilisateur un état de continence, et qu'une tension appliquée par l'utilisateur à l'élément d'extension (32) soit conçue pour relâcher la coaptation de l'urètre et permettre à l'utilisateur d'uriner.

7. Dispositif de traitement de l'incontinence selon la revendication 1, dans lequel le support non poreux (22) possède trois bras comprenant la première partie terminale (28) dotée de la fente (36), la seconde partie terminale (30), et l'élément d'extension (36) qui est fixé au support (22) au niveau de la jonction (34).

8. Dispositif de traitement de l'incontinence selon la revendication 1, dans lequel l'élément d'extension (36) est fixé selon un angle orthogonal au support (22).

9. Dispositif de traitement de l'incontinence selon la revendication 1, dans lequel le manchon (40) est revêtu d'une substance qui est conçue pour empêcher qu'un tissu attache au manchon (40).

10. Dispositif de traitement de l'incontinence selon la revendication 1, dans lequel le support non poreux (22) comprend un premier côté espacé d'un second côté par une largeur de feuille, et la première partie terminale (28) possède une largeur qui est égale à la largeur de feuille et la seconde partie terminale (30) s'effile vers une largeur décroissante qui est inférieure à la largeur de feuille.
